# EUROPEAN PATENT SPECIFICATION

(11) **EP 1 762 227 B1**
(45) Date of publication and mention of the grant of the patent: **27.05.2009**
(21) Application number: 06254698.1
(22) Date of filing: 08.09.2006
(51) Int. Cl.: A61K 9/08, A61P 17/00

(54) **Compositions, uses and kits for treating allergic dermatitis of skin**
Zusammensetzungen, Verwendungen und Kit für die Behandlung von Dermatitis
Compositions, utilisations et kit pour le traitment de la dermatite

(30) Priority: 09.09.2005 US 715647 P; 16.08.2006 US 464986
(43) Date of publication of application: 14.03.2007
(73) Proprietor: Johnson and Johnson Consumer Companies, Inc., Skillman, NJ 08558 (US)
(72) Inventor: Magee, Laura, Milltown, NJ 08850 (US)
(74) Representative: James, Anthony Christopher W.P.

(56) References cited:
- WO-A-20/04052358
- US-A- 4 178 373
- US-A- 6 113 933
- US-A1- 2005 100 621

## Description

### FIELD OF THE INVENTION

The present invention relates to compositions suitable for use in treatments for allergic dermatitis, e.g. Toxicodendron dermatitis resulting from contact with the Rhus oleoresin urushiol.

### BACKGROUND OF THE INVENTION

Urushiol is the toxin responsible for the allergic dermatitis caused by contact with the sap of commonly encountered noxious plants such as poison ivy, poison oak, poison sumac, and related plants found throughout the world. Chemically, urushiols are mixtures of catechols with long, hydrophobic, carbon(alkyl) side chains at the three position of the catechol ring. For example, poison ivy contains predominantly 3-n pentadecylcatechols (C-15) and poison oak contains predominantly 3-n-heptadecylcatechols (C-17).

When skin is exposed to the oleoresin containing the urushiol, the reaction is an allergic eczematous contact dermatitis characterized by redness, swelling, papules, vesicles, bullac and streaking. Treatment of the reaction has historically consisted of attempting to remove the oil as quickly after exposure as possible. If the oil is not removed within a short period of time of exposure, e.g. a matter of a few minutes, attempts to remove the toxin thereafter may not be successful in preventing the allergic reaction, although it may limit its spread.

Attempts have been made to find both prophylactic treatments as well as post-exposure treatments. For example, treatment for poison ivy using a composition that includes linseed oil, an astringent, a starch, an essential oil and citrus oil are known. However, linseed oil can cause irritation to the skin itself. In addition, the composition must be applied to the effected areas up to twice a day until the rash is gone.

US-B-6113933 describes rinse-off antimicrobial cleansing compositions comprising an antimicrobial agent and one or more anionic surfactants. A large number of surfactants and surfactant combinations is disclosed.

Other known treatment for dermatitis caused by exposure to the toxin urushiol includes application of compositions that contain a mixture of nonyl phenyl ethoxylates, followed by removal of the composition from the skin to remove urushiol. In particular, WO-A-2004052358 describes compositions comprising sodium lauroyl sarcosinate and a nonyl phenyl ethoxylate, in combination, for the removal of urushiol from skin,.

However, there are environmental concerns due to the presence of nonylphenols. In addition, in such methods of treatment, the composition is removed within a relatively short period of time and may require multiple applications of the composition. In addition, such treatments leave no therapeutically effective active ingredients on the skin to continue treatment of the allergic reaction.

It would be advantageous to provide a composition that is suitable for the removal of urushiol from the skin and that is more environmentally friendly. The present invention provides environmentally acceptable compositions for the removal of urishiol from skin and kits for use in the treatment of allergic dermatitis caused by exposure to urushiol.

In a first aspect, the present invention provides a composition suitable for removal of urushiol from an area of skin having been exposed thereto, comprising: from 3 to 10 weight percent based on total weight of said composition of an alcohol ethoxylate, wherein said alcohol ethoxylate comprises a C₉-C₁₅ alcohol ethoxylate having HLB of from 9 to 11; and from 2 to 15 weight percent based on total weight of said composition of sodium lauryl sarcosinate; and wherein said alcohol ethoxylate and said sodium lauryl sarcosinate are present in relative amounts effective to remove said urushiol from said skin, and said composition comprises less than 1 weight percent of nonyl phenyl ethoxylates.

In a second aspect, the present invention provides a first composition according to the invention as defined above, for use in a method for treating allergic dermatitis in an area of skin, said method comprising the steps of: contacting an area of skin having been exposed to urushiol with said first composition under conditions effective to remove at least a portion of urushiol from said skin; removing said first composition and said portion of urushiol from said skin; and contacting said exposed area of skin with a second composition comprising a therapeutically effective amount of an active ingredient suitable for treating allergic dermatitis.

In a further aspect, the present invention provides a kit suitable for treatment of allergic dermatitis in an area of skin, comprising: a first composition according to the present invention as defined above; and a second composition comprising a therapeutically effective amount of an active ingredient suitable for treating allergic dermatitis.

The composition may be applied topically and may be delivered by any means know to those skilled in the art for applying topical medicaments, such as lotions, sprays, gels, or articles such as wipes or pads. The composition may be worked over the affected area in a scrubbing motion. The composition remains in contact with the skin area for a period of time effective to remove at least a first portion of the urushiol from the affected area of skin. The time period may range from a few minutes, e.g. 3 minutes, to an hour, or longer, although it is preferred to remove the composition as soon as possible to avoid prolonged exposure of the skin to the urushiol. After sufficient time has elapsed to ensure that the effected skin area has been adequately exposed to the composition, at least a portion of the composition and bound urushiol may be removed, for example by washing the skin.

While complete removal of the composition and bound urushiol is desired, depending on the amount of time and extent of scrubbing one spends in cleaning the effected area of skin, it is probable that in many cases, if not most cases, not all of the urushiol will be removed from the affected area of skin by cleansing with the first composition. Therefore, the affected area of skin is further contacted with a second composition comprising a therapeutically effective amount of an active ingredient suitable for treating allergic contact dermatitis caused by the urushiol. The second composition is applied topically and may be delivered by any means know to those skilled in the art for applying topical medicaments, such as lotions, sprays or gels. The second composition may remain on the effected area for extended periods of time, for instance 1 to 24 hours. The second composition may be applied more than once in order to provide sustained treatment of the effected area.

Preferably, the composition will be free of nonyl phenyl ethoxylates. The composition comprises from 3 to 10 weight percent of the alcohol ethoxylate, and from 2 to 15 weight percent of SLS. One alcohol ethoxylate used is Brij 30^{®}, a C₁₂ alcohol ethoxylate having a HLB of about 10, available from Uniqema, also know as Laureth-4^{®} under CTFA/INCI nomenclature.

The compositions also may comprise an abrasive material to enhance removal of urushiol from exposed skin. The abrasive material assists in the physical detachment of urushiol from the skin and places it in position to more readily bond with the ethoxylate and SLS components of the composition. The beads should be large enough to be effective but not so large as to cause abrasions. In certain embodiments, beads in the range of 5 to 50 microns with an average size being approximately 25 microns, or 50 mesh, may be used. Compositions may comprise from about 5 to about 20 weight percent of the abrasive. Abrasives that may be used include polyethylene beads such as Inducos 13/4^{®}, available from InduChem, and microcrystalline wax beads such as Metabeads Microwax Green 28/60, available from Floratech. One skilled in the art will recognize that alternative abrasive materials may be used in the present invention.

Compositions of the present invention may further comprise thickeners, chelating agents, preservatives, emollients, opacifyng agents, pH buffers and colorants, each in amounts effective to provide their respective functional property. The thickener may comprise an alkyl acrylate polymer, for instance Carbopol ETD^{®} 2020, available from Noveon. The chelating agent may comprise tetrasodium EDTA. Preservative may include methylparaben and phenoxyethanol. The balance of the composition may comprise water.

The second composition used in methods of the present invention may be aqueous based or non-aqueous based. In one composition, an external analgesic, e.g. pramoxine HCL, a skin conditioning agent, e.g. Di-PPG-2 Myreth-10 Adipate, an astringent, e.g. zinc acetate and a denaturant, e.g. t-butyl alcohol and denatonium benzoate, are combined with ethylene alcohol and a film former, e.g. isobutylene/ethylmaleimide/hydroxyethylmaleimide copolymer in water and alcohol, to provide a composition suitable for treatment of allergic dermatitis caused by urushiol. The composition may comprise about 78 weight percent ethyl alcohol, about 20 weight percent film former (comprising about 8% copolymer, about 5% water and about 7% alcohol), about 1 weight percent external analgesic, about 0.75 weight percent skin conditioning agent, about 0.12 weight percent astringent, and about 0.1 weight percent denaturant, each based on the total weight of the composition. Other compositions including active ingredients suitable for treating allergic dermatitis also may be utilized.

The present invention also includes a kit as defined in the accompanying claims suitable for treatment of allergic dermatitis in an area of skin having been exposed to urushiol. The kit comprises a first composition comprising an ethoxylate and sodium lauryl sarcosinate, as described herein above, in relative amounts effective to remove urushiol from an area of skin having been exposed to the urushiol; and a second composition comprising a therapeutically effective amount of an active ingredient suitable for treating allergic dermatitis, as described herein above. The kit may contain the first and second compositions within a single packaging element, or may include the first and second compositions in separate packaging elements. The first and second compositions in said kits may comprise lotions, gels or sprays. The kits also may include articles for applying the first and second compositions to skin, such as wipes containing either of the compositions. In particular, such wipes may include incorporated therewith amounts of the first composition that are effective to remove urushiol from skin, such that additional scrubbing of skin may be accomplished by the use of such wipes, thus providing removal of urushiol from skin. The compositions may be incorporated with the wipes by, for example, spraying or dipping.

The following examples are provided as examples of compositions of the present invention and/or useful in the methods and kits of the present invention and are not intended to limit in any way the scope of the claims appended hereto.

### Example 1:

| Ingredient | Formula % (w/w) |
|---|---|
| Water | 78.6 |
| Glycerin | 2.0 |
| Sodium Lauroyl Sarcosinate | 11.7 |
| Laureth-4 | 5.0 |
| Tetrasodium EDTA | 0.5 |
| DMDMH | 0.9 |
| Methylparaben | 0.25 |
| Active organics | 0.1 |
| Citric Acid (20% sol.) | 0.68 |

A standard wipe was placed in an unsealed packet and the composition of Example 1 was applied to the standard wipe contained within the packet. The packet was then sealed for subsequent use. The composition may be applied to any wipe, pad or other applicator suitable for use in the described application.

### Example 2:

| | |
|---|---|
| Acrylates/C₁₀₋₃₀ Alkyl Acrylate Crosspolymer | 0.85 |
| Glycol Distearate | 1.25 |
| Coco-Glucoside | 0.5 |
| Glyceryl oleate | 0.15 |
| Glycerol stearate | 0.15 |
| Benzoic acid | 0.025 |
| Laureth-4 | 5.0 |
| Sodium Lauroyl Sarcosinate | 2.0 |
| Dowicil | 0.05 |
| Tetrasodium EDTA | 0.19 |
| Sodium hydroxide | 0.0005 |
| Polyethylene | 10.0 |
| Microcrystalline wax | 0.19 |
| Chromium Hydroxide Green | 0.005 |
| Tocopherol | 0.0002 |
| Water | QS |

### Test Methodology:

Two 1 cm diameter test sites were outlined on the volar aspect of each forearm of a subject, for a total of four test sites. Ten microliters of a 1:50 dilution of poison oak/poison ivy oleoresin were applied with disposable micropipettes to 0.6 cm diameter discs of filter paper. The discs were left open to the air for 30 minutes and then carefully applied with forceps to each of the four test sites. The oleoresin-loaded filter paper discs were then covered with occlusive tape (Blenderm, 3M) and left in place for fifteen minutes. After 15 minutes, all of the poison ivy patches were removed and discarded. Sebutapes were applied to one site per forearm of each subject, allowed to remain for 60 seconds and then removed from each of the two sites.

Each of the remaining two test sites was washed with the composition of Example 2 according to the standard instructions. The sites were allowed to air dry for approximately 15 minutes. Sebutapes were then applied to the final two sites, allowed to remain for 60 seconds and then removed.

The Sebutapes were placed in labeled containers and then were extracted in 500 microliters of absolute ethanol. The ethanol samples were then spectrophotometrically analyzed for oleoresins using a Flurorolog Spectrophotometer. The results are shown in Table 1 below.

**Table 1**

| | Sample | % Oleoresin |
|---|---|---|
| Remaining After Washing | | |
| | Control (no washing) | 100 |
| | Composition of Example 1 | 5 |
| | Composition of Example 1 on a wipe | 6 |

The data above demonstrates that the compositions of the present invention are effective at removing oleoresins from the skin.

| Example 3: | Formula % (w/w) |
|---|---|
| Ethyl alcohol | 75 |
| Isobutylene/Ethylmaleimide/ | 8 |
| Hydroxyethylmaleimide Copolymer | |
| Pramoxine HCL | 1.0 |
| Di-PPG-2 Myreth-10 Adipate | 0.75 |
| Zinc Acetate | 0.12 |
| t-butyl alcohol | 0.08 |
| Water | QS |

Compositions as described in Example 3 may be applied to each site of an individual that has been contacted with the oleoresins and washed with compositions of the present invention as described in Examples 1 and 3. Such application will provide ongoing treatment of skin areas having been contacted with the oleoresins.

## Claims

1. A composition suitable for removal of urushiol from an area of skin having been exposed thereto, comprising:
from 3 to 10 weight percent based on total weight of said composition of an alcohol ethoxylate, wherein said alcohol ethoxylate comprises a C₉-C₁₅ alcohol ethoxylate having HLB of from 9 to 11; and
from 2 to 15 weight percent based on total weight of said composition of sodium lauryl sarcosinate; and
wherein said alcohol ethoxylate and said sodium lauryl sarcosinate are present in relative amounts effective to remove said urushiol from said skin, and said composition comprises less than 1 weight percent of nonyl phenyl ethoxylates.

2. The composition of claim 1 comprising about 5 weight percent of a C₁₂ alcohol ethoxylate and about 12 weight percent of said sodium lauryl sarcosinate, said composition being free of said nonyl phenyl ethoxylates.

3. A first composition according to any preceding claim for use in a method for treating allergic dermatitis in an area of skin, said method comprising the steps of:
contacting an area of skin having been exposed to urushiol with said first composition under conditions effective to remove at least a portion of urushiol from said skin;
removing said first composition and said portion of urushiol from said skin; and
contacting said exposed area of skin with a second composition comprising a therapeutically effective amount of an active ingredient suitable for treating allergic dermatitis.

4. The first composition of claim 3 wherein said active ingredient comprises an analgesic.

5. The first composition of claim 3 wherein said second composition remains in contact with said skin for a time of from about 1 to about 24 hours.

6. The first composition of claim 3 wherein said second composition is applied more than once.

7. The first composition of claim 3 wherein said first composition is applied to said skin by a lotion, spray, gel or wipe.

8. A kit suitable for treatment of allergic dermatitis in an area of skin, comprising:
a first composition according to claim 1 or 2; and
a second composition comprising a therapeutically effective amount of an active ingredient suitable for treating allergic dermatitis.

9. The kit of claim 8 wherein said active ingredient comprises an analgesic.

10. The kit of claim 8 comprising an article having incorporated therewith said first composition.

## Patentansprüche

1. Zusammensetzung, die zur Entfernung von Urushiol von einer Hautfläche, die diesem ausgesetzt gewesen ist, geeignet ist, umfassend:
von 3 bis 10 Gewichtsprozent, bezogen auf das Gesamtgewicht der Zusammensetzung, eines Alkoholethoxylats, wobei das Alkoholethoxylat ein C₉-C₁₅-Alkoholethoxylat mit einem HLB von 9 bis 11 umfasst; und
von 2 bis 15 Gewichtsprozent, bezogen auf das Gesamtgewicht der Zusammensetzung, Natriumlaurylsarcosinat; und
wobei das Alkoholethoxylat und das Natriumlaurylsarcosinat in relativen Mengen vorliegen, die darin wirksam, das Urushiol von der Haut zu entfernen, und die Zusammensetzung weniger als 1 Gewichtsprozent Nonylphenylethoxylate umfasst.

2. Zusammensetzung nach Anspruch 1, die etwa 5 Gewichtsprozent eines C₁₂-Alkoholethoxylats und etwa 12 Gewichtsprozent des Natriumlaurylsarcosinats umfasst, wobei die Zusammensetzung frei von Nonylphenylethoxylaten ist.

3. Erste Zusammensetzung nach einem vorangehenden Anspruch zur Verwendung in einem Verfahren zur Behandlung allergischer Dermatitis auf einer Hautfläche, wobei das Verfahren die Schritte umfasst:
Inkontaktbringen einer Hautfläche, die Urushiol ausgesetzt gewesen ist, mit der ersten Zusammensetzung unter Bedingungen, die darin wirksam sind, wenigstens einen Teil des Urushiols von der Haut zu entfernen;
Entfernen der ersten Zusammensetzung und des Teil des Urushiols von der Haut; und
Inkontaktbringen der exponierten Hautfläche mit einer zweiten Zusammensetzung, die eine therapeutisch wirksame Menge eines Wirkstoffes umfasst, der zur Behandlung allergischer Dermatitis geeignet ist.

4. Erste Zusammensetzung nach Anspruch 3, wobei der Wirkstoff ein Analgetikum umfasst.

5. Erste Zusammensetzung nach Anspruch 3, wobei die zweite Zusammensetzung mit der Haut für einen Zeitraum von etwa 1 bis etwa 24 Stunden in Kontakt bleibt.

6. Erste Zusammensetzung nach Anspruch 3, wobei die zweite Zusammensetzung mehr als einmal aufgebracht wird.

7. Erste Zusammensetzung nach Anspruch 3, wobei die erste Zusammensetzung auf die Haut mit einer Lotion, einem Spray, einem Gel oder einem Tuch aufgebracht wird.

8. Kit, der zur Behandlung allergischer Dermatitis auf einer Hautfläche geeignet ist, umfassend:
eine erste Zusammensetzung nach Anspruch 1 oder 2; und
eine zweite Zusammensetzung, die eine therapeutisch wirksame Menge eines Wirkstoffes umfasst, der zur Behandlung allergischer Dermatitis geeignet ist.

9. Kit nach Anspruch 8, wobei der Wirkstoff ein Analgetikum umfasst.

10. Kit nach Anspruch 8, der einen Gegenstand umfasst, in den die erste Zusammensetzung eingearbeitet ist.

## Revendications

1. Composition appropriée pour éliminer l'urushiol d'une zone de la peau ayant été exposée à celui-ci, comprenant :
► de 3 à 10 pour cent en poids du poids total de ladite composition d'un éthoxylat d'alcool, où ledit éthoxylat d'alcool comprend un éthoxylat d'alcool en C₉ à C₁₅ ayant un indice HLB de 9 à 11 ; et
► de 2 à 15 pour cent en poids du poids total de ladite composition de laurylsarcosinate de sodium ; et
► où ledit éthoxylat d'alcool et ledit laurylsarcosinate de sodium sont présents en des quantités relatives efficaces pour éliminer ledit urushiol de ladite peau, et ladite composition comprend moins de 1 pour cent en poids d'éthoxylats de nonylphényle.

2. Composition selon la revendication 1, comprenant environ 5 pour cent en poids d'un éthoxylat d'alcool en C₁₂ et environ 12 pour cent en poids dudit laurylsarcosinate de sodium, ladite composition étant exempte desdits éthoxylats de nonylphényle.

3. Première composition selon l'une quelconque des revendications précédentes, pour une utilisation dans un procédé pour le traitement de la dermatite allergique dans une zone de la peau, ledit procédé comprenant les étapes consistant à :
► mettre en contact une zone de la peau ayant été exposée à l'urushiol ave.c ladite première composition dans des conditions efficaces pour éliminer au moins une partie de l'urushiol de ladite peau ;
► éliminer ladite première composition et ladite partie d'urushiol de ladite peau ; et
► mettre en contact ladite zone de peau exposée avec une seconde composition comprenant une quantité thérapeutiquement efficace d'un ingrédient actif approprié pour traiter la dermatite allergique.

4. Première composition selon la revendication 3, dans laquelle ledit ingrédient actif comprend un analgésique.

5. Première composition selon la revendication 3, dans laquelle ladite seconde composition reste en contact avec ladite peau pendant une durée d'environ 1 à environ 24 heures.

6. Première composition selon la revendication 3, dans laquelle ladite seconde composition est appliquée plusieurs fois.

7. Première composition selon la revendication 3, dans laquelle ladite première composition est appliquée à ladite peau par une lotion, un spray, un gel ou une lingette.

8. Nécessaire approprié pour traiter la dermatite allergique dans une zone de la peau, comprenant :
► une première composition selon la revendication 1 ou 2 ; et
► une seconde composition comprenant une quantité thérapeutiquement efficace d'un ingrédient actif approprié pour traiter la dermatite allergique.

9. Nécessaire selon la revendication 8, dans lequel ledit ingrédient actif comprend un analgésique.

10. Nécessaire selon la revendication 8, comprenant un article dans lequel est incorporée ladite première composition.
